# EUROPEAN PATENT SPECIFICATION

(11) **EP 3 612 173 B1**
(45) Date of publication and mention of the grant of the patent: **11.10.2023**
(21) Application number: 18786961.5
(22) Date of filing: 17.04.2018
(51) Int. Cl.: A61K 9/08, A61K 47/18, A61K 47/02, A61K 47/10, A61K 31/137, A61P 37/08

(54) **EPINEPHRINE SPRAY FORMULATIONS**
EPINEPHRINSPRÜHFORMULIERUNGEN
FORMULATIONS POUR LA PULVÉRISATION D'ÉPINÉPHRINE

(30) Priority: 17.04.2017 US 201715488712
(43) Date of publication of application: 26.02.2020
(73) Proprietor: Hikma Pharmaceuticals USA Inc., Eatontown, New Jersey 07724-2206 (US)
(72) Inventor: POTTA, Thrimoorthy, Phoenix AZ 85048 (US); BASTIAN, Craig, Mesa AZ 85212 (US); YAN, Ningxin, Chandler AZ 85249 (US); GOSKONDA, Venkat, Phoenix AZ 85044 (US); CHILAMPALLI, Chandeshwari, Chandler AZ 85249 (US); INAVOLU, Rachana, Chandler AZ 85286 (US); NARAYANAN, Eshwaran, Chandler AZ 85225 (US)
(74) Representative: Elkington and Fife LLP
(86) International application number: PCT/US2018/027889
(87) International publication number: WO 2018/195029

(56) References cited:
- US-A- 3 705 942
- US-A1- 2007 059 361
- US-A1- 2014 162 965
- US-A1- 2015 005 356
- US-A1- 2015 246 009
- US-A1- 2017 079 907
- US-A1- 2017 079 907
- US-A1- 2017 216 199
- US-B2- 7 947 742

## Description

### FIELD OF THE INVENTION

The present invention is directed to epinephrine spray formulations for use in methods of treating anaphylaxis by administering epinephrine spray formulations to subjects in need of such treatments.

### BACKGROUND OF THE INVENTION

Epinephrine (i.e. adrenaline) is a catecholamine with the following chemical structure:

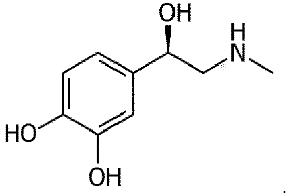

Epinephrine stimulates the alpha- and beta- adrenergic receptors of the sympathetic nervous system. Epinephrine binds to these adrenergic receptors leading to relief of many life-threatening symptoms of anaphylaxis including: relaxation of the smooth muscle in the bronchi of the lungs opening up the constricted airways; constriction of the blood vessels leading to decreased swelling of the tongue and throat and increased blood pressure; and finally, increased heart rate preventing or reversing cardiovascular collapse.

Epinephrine is commercially available as an injection (Adrenalin^{®} a trademark of and available from Par Sterile Products, LLC) and an auto-injector (EpiPen^{®} a trademark of and available from Mylan, Inc. and Auvi-Q^{®} a trademark of and available from Sanofi Corporation). Epinephrine was previously available as a nasal spray (Adrenalin^{®}) and an aerosol spray (Primatene^{®} Mist trademark of Armstrong Pharmaceuticals, Inc.). Racepinephrine is commercially available as a 2.25% oral inhalation solution for use in nebulizers (S2^{®} is available from Nephron Pharmaceuticals, Inc.). Epinephrine differs from racepinephrine in that epinephrine consists of only the L-isomer and racepinephrine is a 50/50 mixture of both the Land D-isomers.

U.S. Patent No. 8,628,805 is directed to a stable liquid adrenaline/bisulfite composition wherein the molar ratio of adrenaline to bisulfite is 1.31-2.20:1. U.S. Patent Application Publication No. 2012/0322884 A1 is directed to epinephrine nanoparticles, which can be incorporated into sublingual tablets. U.S. Patent Application Publication No. 2007/0202163 A1 is directed to epinephrine tablets for sublingual administration, which contain between 12% and 48% epinephrine. World Intellectual Property Organization ("W.I.P.O.") Publication No. 2014/127018 A1 is directed to a stable aqueous epinephrine formulation that requires cyclodextrin. W.I.P.O. Publication No. 2014/057365 A1 is directed to an injectable epinephrine formulation. U.S. Patent Application Publication No. 2017/0079907 is directed to sublingual epinephrine spray formulations, and further directed to methods of treating anaphylaxis by administering sublingual epinephrine spray formulations to subjects in need of such treatment. U.S. Patent Application Publication No. 2015/0005356 is directed to pharmaceutical compositions of epinephrine for delivery to the nasal mucosa and methods of treating a subject in acute severe anaphylaxis, bronchospasm or during cardiopulmonary resuscitation (CPR). The composition further comprising agents, that either prevent localized degradation of epinephrine or enhance its absorption in the nasal mucosa to counter anaphylactic effects, symptoms or complications in a subject. U.S. Patent No. 3,705,942 is directed to the treatment of glaucoma with compositions containing methylimipramine or imipraminie optionally in the presence of an alkyl gallate and/or a sympathetic or sympathomimetic amine. A typical embodiment is an ophthalmic pharmaceutical composition containing desmethylimipramine and epinephrine.

While there are various epinephrine formulations currently available, there remains a need in the art for a quick-onset spray formulation.

### SUMMARY OF THE INVENTION

In one aspect, the present invention is directed to epinephrine spray formulations for use in methods of treating anaphylaxis, where in the formulations comprise:
from about 0.1% w/w to about 15% w/w epinephrine or a salt thereof, preferably the salt is selected from the group consisting of citrate, hydrochloride, halide, sulfate, phosphate, acetate, maleate, succinate, ascorbate, carbonate, mesylate and lactate;
from about 1 % to about 65 % w/w hydrochloric acid with a normality from about 0.1 to 12N;
from about 2 % to about 60 % w/w ethanol;
from about 2% to about 98 % w/w water;
from about 0.001 % to about 0.5 % w/w sodium bisulfite;
from about 0.005 % to about 1 % w/w edetate disodium dihydrate and,
from about 0.001% to about 0.5% w/w benzalkonium chloride; and
wherein the formulation is administered via the intranasal route.

In another aspect, the present invention is directed to epinephrine spray formulations, wherein the formulation is free of a propellant.

In another aspect, the solvent may comprise from about 1 % w/w to about 30% w/w glycerin.

In another aspect, the epinephrine spray formulations further comprise a permeation enhancer selected from the group consisting of caprylic acid, menthol, limonene, carvone, methyl chitosan, polysorbates, sodium lauryl sulfate, glyceryl oleate, caproic acid, enanthic acid, pelargonic acid, capric acid, undecylenic acid, lauric acid, myristic acid, palmitic acid, oleic acid, stearic acid, linolenic acid, arachidonic acid, benzethonium chloride, benzethonium bromide, benzalkonium chloride (BKC), cetylpyridium chloride, edetate disodium dihydrate, sodium desoxycholate, sodium deoxyglycolate, sodium glycocholate, sodium caprate, sodium taurocholate, sodium hydroxybenzoyal amino caprylate, dodecyl dimethyl aminopropionate, L-lysine, glycerol oleate, glyceryl monostearate, citric acid, peppermint oil and a combination thereof, preferably a combination of edetate disodium dihydrate and BKC. The epinephrine formulations according to the invention comprise from about 0.005% to about 1 % w/w edetate disodium dihydrate and from about 0.001% to about 0.5% w/w benzalkonium chloride, preferably the edetate disodium dihydrate is from about 0.005% to about 0.05% w/w and the benzalkonium chloride is preferably at a concentration from about 0.005% to about 0.5% w/w

In another aspect, permeation enhancer comprises caprylic acid, preferably the caprylic acid is at a concentration from about 0.1% w/w to about 10 % w/w, more preferably from about 0.1% w/w to about 5 % w/w.

In another aspect, the epinephrine spray formulations contain no additional permeation enhancer.

In another aspect, the epinephrine spray formulations can further comprise an isotonicity agent comprising sodium chloride.

In another aspect, the epinephrine spray formulations further comprise a stabilizer selected from the group consisting of butylated hydroxyanisole (BHA), butylated hydroxytoluene (BHT), ascorbic acid, methionine, sodium ascorbate, sodium thiosulfate, sodium bisulfite, sodium metabisulfite, ascorbyl palmitate, thioglycerol, alpha tocopherol (vitamin E), cysteine hydrochloride, benzalkonium chloride, citric acid, ethylenediaminetetraacetic acid (EDTA), sodium citrate, propyl gallate, 8-hydroxyquinoline, boric acid, histidine and combinations thereof.

The epinephrine formulations according to the invention comprise from about 0.001 % to about 0.5 % w/w sodium bisulfite and from about 0.005 % to about 1 % w/w edetate disodium dihydrate.

In another aspect, the epinephrine spray formulations further comprise a preservative selected from the group consisting of butyl paraben, methyl paraben, ethyl paraben, propyl paraben, sodium benzoate, chlorobutanol, benzalkonium chloride (BKC), benzoic acid and combinations thereof. The epinephrine formulations of the present invention comprise from about 0.001% to about 0.5% w/w BKC, preferably BKC at a concentration from about 0.005% w/w to about 0.5% w/w.

The present invention is directed to epinephrine spray formulations comprising:
from about 0.1 % to about 15 % w/w epinephrine, or a salt thereof;
from about 1 % to about 65 % w/w hydrochloric acid with a normality from about 0.1 to 12N;
from about 2 % to about 60 % w/w ethanol;
from about 2% to about 98 % w/w water;
from about 0.001 % to about 0.5 % w/w sodium bisulfite;
from about 0.005 % to about 1 % w/w edetate disodium dihydrate; and
from about 0.001% to about 0.5% w/w benzalkonium chloride.

In another aspect, the present invention is directed to an epinephrine spray formulation comprising:
about 3.2 % w/w epinephrine, or a salt thereof;
about 35.5 % w/w hydrochloric acid with a normality of 0.5;
about 40 % w/w ethanol;
about 16.1 % w/w water;
about 0.05 % w/w edetate disodium dihydrate;
about 0.15 % w/w sodium bisulfite;
about 0.01 % w/w benzalkonium chloride;
wherein % w/w denotes weight by total weight of the formulation.

In another aspect, the present invention is directed to an epinephrine spray formulation of the present invention for use in a method of treating anaphylaxis, wherein administration occurs via the intranasal route.

### DETAILED DESCRIPTION OF THE INVENTION

### Definitions

Applicants discovered epinephrine spray formulations that have improved bioavailability, a more rapid onset of action, and improved storage stability.

As used herein, "epinephrine" refers to the base.

As used herein, "free of propellant" refers to a formulation that is not administered using compressed gas.

As used herein, all numerical values relating to amounts, weights, and the like, that are defined as "about" each particular value is plus or minus 10%. For example, the phrase "about 10% w/w" is to be understood as "9% w/w to 11% w/w." Therefore, amounts within 10% of the claimed value are encompassed by the scope of the claims.

As used herein "% w/w" and "percent w/w" refer to the percent weight of the total formulation.

As used herein the term "effective amount" refers to the amount necessary to treat a patient in need thereof.

As used herein the term "treat", "treating" or "treatment" refers to ameliorating or inhibiting symptoms of type I allergies including anaphylaxis.

As used herein the term "subject" refers, but is not limited to, a person that is experiencing type I allergies including anaphylaxis.

As used herein the term "anaphylaxis" refers to an allergic reaction involving multiple organ systems in a subject upon contact with an allergen rather or not that allergen is identifiable.

As used herein the term "allergen" refers to any chemical capable of causing an immune system response in a subject including, but not limited to, chemicals found in drugs, food, plants, insect bites, and insect stings.

As used herein the term "pharmaceutically acceptable" refers to ingredients that are not biologically or otherwise undesirable for administration to a living subject.

"Sublingual" refers to administration of a substance via the mouth in such a way that the substance is rapidly absorbed via the blood vessels under the tongue.

"Intranasal" refers to administration of the composition to any portion of the nasal epithelium.

The present invention is directed to epinephrine spray formulations comprising epinephrine, or a salt thereof, for use in a method of treating anaphylaxis.

Preferred epinephrine salts include citrate, hydrochloride, halide, sulfate, tartrate, phosphate, acetate, malate, maleate, succinate, ascorbate, carbonate, mesylate and lactate. One of skill in the art could use other pharmaceutically acceptable epinephrine salts in the formulations of the present invention. The formulations contain epinephrine or the pharmaceutically acceptable salt equivalent to from about 0.1% to about 15 % w/w epinephrine. In a more preferred embodiment, the formulation contains epinephrine or the pharmaceutically acceptable salt equivalent to from about 0.1 % to about 10 % w/w of epinephrine. Other most preferred embodiments include formulations which contain epinephrine or the pharmaceutically acceptable salt equivalent to from about 0.1 % w/w to about 8 % w/w, from about 0.1 % to about 6.5 % w/w. In a most preferred embodiment, the epinephrine concentration is 3% and 6% w/w.

In another embodiment, the present invention is directed to epinephrine spray formulations comprising epinephrine, or a salt thereof, wherein the formulation is free of a propellant, for use in a method of treating anaphylaxis.

In another embodiment, the present invention is directed to epinephrine spray formulations comprising epinephrine, or a salt thereof, and one or more excipients selected from acids, solvents, stabilizers, permeation enhancers, viscosity modifiers, sweeteners, sweetness enhancers, pH modifiers, isotonicity agents and flavoring agents, for use in a method of treating anaphylaxis.

The formulations of the present invention contain from about 1% to about 65 % w/w hydrochloric acid with a normality from about 0.1N to 12N preferably 0.5N-3N hydrochloric acid, and more preferably 0.5 N or 1 N.

In a preferred embodiment, the formulations contain from about 1% to 99% w/w of a solvent preferably from about 30% to about 99% w/w of the solvent.

Solvents suitable for use in the present invention include, but are not limited to, water, ethanol, glycerin, propylene glycol, polyethylene glycol 400 and combinations thereof, more preferably water. The formulations of the present invention comprise from about 2 % to about 60 % w/w ethanol and from about 2 % to about 98 % w/w water.

In a preferred embodiment, the formulations contain from about 0.001% to about 10% w/w of a stabilizer, preferably from about 0.005 % to about 7.5 % w/w, and even more preferably from about 0.01% to about 5% w/w. Stabilizers suitable for use in the present invention include, but are not limited to, butylated hydroxyanisole (BHA), butylated hydroxytoluene (BHT), ascorbic acid, methionine, sodium ascorbate, sodium thiosulfate, sodium bisulfite, sodium metabisulfite, ascorbyl palmitate, thioglycerol, alpha tocopherol (vitamin E), cysteine hydrochloride, citric acid, ethylenediaminetetraacetic acid ("EDTA"), sodium citrate, propyl gallate, 8-hydroxyquinoline, boric acid, histidine and combinations thereof. In a preferred embodiment, the stabilizer is selected from sodium metabisulfite, sodium bisulfite, EDTA, 8-hydroxyquinoline and combinations thereof. In an even more preferred embodiment the stabilizer is a combination of sodium bisulfite, sodium metabisulfite, 8-Hydroxyquinoline and EDTA. In a further preferred embodiment, the formulations of the present invention contain from about 0.005% to 0.5% w/w EDTA as the stabilizer. In a more preferred embodiment, the formulations of the present invention contain from about 0.01% to 0.1% EDTA as the stabilizer. In a most preferred embodiment, the formulations of the present invention contain about 0.05% EDTA as the stabilizer. In a further preferred embodiment, the formulations contain sodium bisulfite and sodium metabisulfite as the anti-oxidants at a concentration from about from about 0.005% to 5% w/w. In a more preferred embodiment, the formulations contain sodium bisulfite or sodium metabisulfite or a combination thereof as the anti-oxidants at a concentration from about from about 0.05% to 1% w/w. In a more preferred embodiment, the formulations contain sodium bisulfite or sodium metabisulfite combination thereof as the anti-oxidants at a concentration from about from about 0.1% to 0.5% w/w. The formulations of the present invention comprise from about 0.001 % to about 0.5 % w/w sodium bisulfite. In a most preferred embodiment, the formulations of the present invention contain sodium bisulfite as the anti-oxidant at a concentration at about 0.15 % or 0.3% or 0.5 % w/w.

In some embodiments, the formulations can contain from about 0.001% w/w to about 15% w/w of a permeation enhancer, preferably from about 0.03 % w/w to about 12 % w/w, and even more preferably from about 0.05% to 10% w/w.

Permeation enhancers suitable for use in the present invention include, but are not limited to, caprylic acid, oleic acid, polysorbate 80, menthol, edetate disodium dihydrate, benzalkonium chloride, EDTA, sodium edetate, cetylpyridinium chloride, sodium lauryl sulfate, citric acid, sodium desoxycholate, sodium deoxyglycolate, glyceryl oleate, L-lysine and combinations thereof. Preferred permeation enhancers are caprylic acid, menthol or a combination thereof.

Viscosity modifiers suitable for the present invention include, but are not limited to, polyvinylpyrrolidone, carboxymethyl cellulose, hydroxypropylmethyl cellulose ("HPMC"), methyl cellulose, hydroxyethyl cellulose, glycerin, polyvinyl alcohol and combinations thereof. In a preferred embodiment, the viscosity modifier is HPMC.

Sweeteners suitable for the present invention include, but are not limited to, sucralose, sucrose, aspartame, saccharin, dextrose, mannitol, glycerin, xylitol and combinations thereof. In a preferred embodiment, the sweetener is sucralose.

In some embodiments, the formulations can contain from about 0.001% to about 1% of a sweetness enhancer. Sweetness enhancers suitable for the present invention include, but are not limited to, the ammonium salt forms of crude and refined Glycyrrhizic Acid. Magnasweet^{®} products (available from Mafco Worldwide Corporation, Magnasweet is a registered trademark of Mafco Worldwide Corporation) use the ammonium salt forms of crude and refined Glycyrrhizic Acid. Glycyrrhizic Acid is also available as a pure derivative in the sodium and potassium salt forms.

In a preferred embodiment, the formulations are at a pH from about 2.0 to about 5.0. In a more preferred embodiment the formulations of the present invention are at a pH from about 3.0 to about 5.0. In a further preferred embodiment, the formulations of the present invention are at a pH from about 4.0 to about 5.0. In a most preferred embodiment the formulations of the present invention are at a pH of 4.5. pH modifiers suitable for the present invention include, but are not limited to, hydrochloric acid, citric acid, fumaric acid, lactic acid, sodium hydroxide, sodium citrate, sodium bicarbonate, sodium carbonate, ammonium carbonate and combinations thereof.

Preservatives suitable for the formulations include, but are not limited to, butyl paraben, methyl paraben, ethyl paraben, propyl paraben, sodium benzoate, chlorobutanol, benzalkonium chloride, benzoic acid and combinations thereof. The formulations according to the invention comprise from about 0.001 % to about 0.5 % w/w benzalkonium chloride. In preferred embodiment the benzalkonium chloride is at a concentration from about 0.01% to about 0.02% w/w, more preferably 0.01% or 0.02% w/w.

Flavoring agents suitable for the present invention include, but are not limited to, peppermint oil, menthol, spearmint oil, citrus oil, cinnamon oil, strawberry flavor, cherry flavor, raspberry flavor, orange oil and a combination thereof.

The present invention is directed to epinephrine spray formulations comprising:
from about 0.1 % to about 15 % w/w epinephrine, or a salt thereof;
from about 1 % to about 65 % w/w hydrochloric acid with a normality from about 0.1 to 12N;
from about 2 % to about 60 % w/w ethanol;
from about 2% to about 98 % w/w water;
from about 0.001 % to about 0.5 % w/w sodium bisulfite;
from about 0.005 % to about 1 % w/w edetate disodium dihydrate; and from about 0.001% to about 0.5% w/w benzalkonium chloride.

In preferred embodiment, the present invention is directed to epinephrine spray formulations comprising:
from about 2.8 % to about 4 % w/w epinephrine, or a salt thereof;
from about 32 % to about 38 % w/w hydrochloric acid with a normality of 0.5;
from about 10 % to about 65 % w/w ethanol;
from about 2 % to about 38 % w/w water;
about 5 % w/w propylene glycol;
from about 0.1 % to about 0.3 % w/w sodium bisulfite;
about 0.05% w/w EDTA;
optionally, a permeation enhancer selected from the group consisting of from about 2 % to about 10 % w/w caprylic acid, from about 0.5 % to about 1.0 % w/w menthol and a combination thereof; and
benzalkonium chloride at a concentration from about 0.01 % to about 0.02 % w/w, wherein the formulation optionally has a pH of about 4.5.

In another preferred embodiment, the present invention is directed to epinephrine spray formulations comprising:
about 3.24 % w/w epinephrine, or a salt thereof;
about 36.2 % w/w hydrochloric acid with a normality of 0.5;
about 40 % w/w ethanol;
about 14.84 % w/w water;
about 5 % w/w propylene glycol;
about 0.01% w/w benzalkonium chloride; and
from about 0.15 % to about 0.3 % w/w sodium bisulfite,
wherein the formulation has a pH at about 4.5.

In another preferred embodiment, the present invention is directed to intranasal epinephrine spray formulations comprising:
about 3.117 % w/w epinephrine, or a salt thereof;
about 34.8 % w/w hydrochloric acid with a normality of 0.5;
about 20 % w/w ethanol;
about 36.87 % w/w water;
about 5 % w/w propylene glycol;
about 0.01% w/w benzalkonium chloride;
from about 0.15 % w/w sodium bisulfite to about 0.3 % w/w sodium bisulfite,
wherein the formulation has a pH at about 4.5.

In another preferred embodiment, the present invention is directed to epinephrine spray formulations comprising:
about 3.178 % w/w epinephrine, or a salt thereof;
about 35.471 % w/w hydrochloric acid with a normality of 0.5;
about 16.141 % w/w water;
about 40 % ethanol;
about 0.05% w/w edetate disodium dihydrate;
about 0.15 % w/w sodium bisulfite; and
about 0.01% w/w benzalkonium chloride.

In another embodiment, the formulations of the present invention are capable of producing a droplet size distribution at 3 cm wherein the mean DV (10) is from about 15 to about 18 microns during administration.

In another embodiment, the formulations of the present invention are capable of producing a droplet size distribution at 3 cm wherein the mean DV (50) is from about 30 to about 34 microns during administration.

In another embodiment, the formulations of the present invention are capable of producing a droplet size distribution at 3 cm wherein the mean DV (90) is from about 120 to about 230 microns during administration.

In another embodiment, the formulations of the present invention are capable of producing a spray span ((Dv90-Dv10)/Dv50) at 3 cm of from about 3 to about 7.

In another embodiment, the formulations of the present invention are capable of producing a droplet size distribution at 6 cm wherein the mean DV (10) is from about 22 to about 25 microns during administration.

In another embodiment, the formulations of the present invention are capable of producing a droplet size distribution at 6 cm wherein the mean DV (50) is from about 36 to about 41 microns during administration.

In another embodiment, the formulations of the present invention are capable of producing a droplet size distribution at 6 cm wherein the mean DV (90) is from about 59 to about 231 microns during administration.

In another embodiment, the formulations of the present invention are capable of producing a spray span ((Dv90-Dv10)/Dv50) at 6 cm of from about 1 to about 6.

In another embodiment, the formulations of the present invention are capable of producing a spray pattern at 3 cm wherein the Dmin is from about 18 to about 23 millimeters during administration.

In another embodiment, the formulations of the present invention are capable of producing a spray pattern at 3 cm wherein the Dmax is from about 29 to about 33 millimeters during administration.

In another embodiment, the formulations of the present invention are capable of producing a spray pattern at 3 cm wherein the ovality ratio is from about 1.4 to about 1.7 during administration.

In another embodiment, the formulations of the present invention are capable of producing a spray pattern at 6 cm wherein the Dmin is from about 26 to about 33 millimeters during administration.

In another embodiment, the formulations of the present invention are capable of producing a spray pattern at 6 cm wherein the Dmax is from about 47 to about 52 millimeters during administration.

In another embodiment, the formulations of the present invention are capable of producing a spray pattern at 6 cm wherein the ovality ratio is from about 1.6 to about 1.9 during administration.

In another embodiment, the formulations of the present invention are capable of producing a plume geometry at 3 cm wherein the angle is from about 49° to about 64°.

In another embodiment, the formulations of the present invention are capable of producing a plume geometry at 3 cm wherein the width is from about 27 to about 38 millimeters.

In another embodiment, the formulations of the present invention are capable of producing a plume geometry at 3 cm wherein the angle is from about 37° to about 44°.

In another embodiment, the formulations of the present invention are capable of producing a plume geometry at 3 cm wherein the width is from about 37 to about 44 millimeters.

The disclosed embodiments are simply exemplary embodiments of the inventive concepts disclosed herein and should not be considered as limiting, unless the claims expressly state otherwise.

The following examples are intended to illustrate the present invention and to teach one of ordinary skill in the art how to use the formulations of the invention. They are not intended to be limiting in any way.

### EXAMPLES

### Reference Example 1

An epinephrine spray was prepared as follows using the components and amounts listed in Table 1 below. All of the solvents were purged with nitrogen prior to use. Excipients including 0.5 N hydrochloric acid ("HCl"), malic acid, ethanol and propylene glycol, EDTA, sodium chloride, sodium bisulfite, sodium metabisulfite, and 8-hydroxyquinoline were dissolved in water while stirring at room temperature. Epinephrine base was then added to the excipient solution. Finally, sodium hydroxide ("NaOH")/ hydrochloric acid (HCl)) was used to adjust final pH.

**Table 1. Epinephrine Formulations**

| % w/w | #1 | #2 | #3 | #4 | #5 | #6 | #7 | #8 | #9 | #10 | #11 |
|---|---|---|---|---|---|---|---|---|---|---|---|
| Epinephrine base | 1.0 | 1.0 | 1.0 | 1.0 | 1.0 | 7.5 | 1 | 3.0 | 3.0 | 3.0 | 3.0 |
| HCl (0.5 N) | 10.66 | 10.66 | 10.66 | 10.66 | 10.66 | - | 11.35 | - | 32.6 | 32.6 | 32.6 |
| EDTA | 0.05 | 0.05 | 0.05 | 0.05 | 0.05 | - | - | - | 0.05 | 0.05 | 0.05 |
| Sodium metabisulfite | 0.01 | 0.015 | 0.02 | 0.025 | 0.025 | 0.25 | 0.025 | 0.04 | - | - | - |
| sodium bisulfite | 0.01 | 0.015 | 0.02 | 0.025 | 0.025 | - | 0.025 | | 0.05 | 0.2 | 0.2 |
| Propylene Glycol | - | - | - | - | - | - | - | - | - | - | 5 |
| Menthol | - | - | - | - | - | - | - | - | - | - | 5 |
| Chlorobutanol | - | - | - | - | - | - | - | - | - | 0.5 | - |
| Sucralose | - | - | - | - | - | - | - | - | - | 0.5 | 0.5 |
| 8-Hydroxyquinoline | - | - | - | - | 0.02 | - | - | - | - | - | - |
| Ethanol | - | - | - | - | - | 15 | | - | - | - | 50 |
| Malic Acid | - | - | - | - | - | 2.0 | - | 1.65 | - | - | - |
| Water | 88.27 | 88.26 | 88.25 | 88.24 | 88.22 | 79.75 | 87.6 | 95.31 | 64.3 | 63.15 | 4.15 |
| | 100 | 100 | 100 | 100 | 100 | 100 | 100 | 100 | 100 | 100 | 100 |
| pH adjusted with NaOH | pH 4.5 | pH 4.5 | pH 4.5 | pH 4.5 | pH 4.5 | pH 4.5 | pH 4.5 | pH 4.5 | pH 4.5 | pH 4.5 | pH 4.5 |

### Reference Example 2

The formulations listed in Table 1 were subjected to stability at 40°C ± 2°C/75 % ± 5 % relative humidity and 25°C ± 2°C/60 % ± 5 % relative humidity. The stability of the formulations was analyzed at specified time points by evaluating their potency (assay value) and impurity levels. Assay and impurities were detected using high-performance liquid chromatography with an ultraviolet detector. The assay was performed at 280 nm and indicated as a % of initial concentration. For all impurities, analysis was performed at 210 nm and 280nm and expressed as a % area. Amounts of particular impurities are listed in Tables 2 to 20 as a percentage of area of each formulation along with amount of total impurities. Relative retention time ("RRT") is given for each impurity. "ND" indicates that the impurity was not detected. "BQL" indicates purity is below quantification limit.

**Table 2. Stability Data for Epinephrine Spray Formulation #1 stored at 40°C ± 2°C/ 75 % ± 5 % Relative Humidity**

| 40 °C Formulation #1 | RRT | 0 Week | 2 Weeks | 4 Weeks |
|---|---|---|---|---|
| Appearance | | Clear | Clear | Clear |
| Assay (% of initial conc.) | | 100.00 | 97.94 | 95.80 |
| % Racemization | | 0.60 | 0.93 | 1.54 |
| pH | | 4.50 | 3.52 | 3.29 |
| % Impurity F | 0.19 | 0.13 | 1.07 | 1.51 |
| % Synephrine | 1.26 | ND | ND | ND |
| % Epinephrone | 1.36 | ND | ND | ND |
| % Methoxy | 1.88 | 0.07 | 0.07 | 0.07 |
| % Unknown Impurity | 0.21 | ND | 0.10 | 0.15 |
| | 0.23 | ND | ND | 0.02 |
| | 3.11 | ND | 0.01 | 0.03 |
| | 3.26 | ND | 0.01 | 0.02 |
| % Total Impurities | | 0.20 | 1.26 | 1.80 |

**Table 3. Stability Data for Epinephrine Spray Formulation #2 stored at 40°C ± 2°C/ 75 % ± 5 % Relative Humidity**

| 40 °C Formulation #2 | RRT | 0 Week | 2 Weeks | 4 Weeks |
|---|---|---|---|---|
| Appearance | | Clear | Clear | Clear |
| Assay (% of initial conc.) | | 100.00 | 96.02 | 97.32 |
| % Racemization | | 0.60 | 0.91 | 1.77 |
| pH | | 4.50 | 3.39 | 3.14 |
| % Impurity F | 0.19 | 0.14 | 1.27 | 1.91 |
| % Synephrine | 1.26 | ND | ND | ND |
| % Epinephrone | 1.38 | ND | ND | 0.01 |
| % Methoxy | 1.88 | 0.07 | 0.07 | 0.07 |
| % Unknown Impurity | 0.21 | ND | 0.10 | 0.11 |
| | 0.23 | ND | ND | 0.01 |
| | 3.26 | ND | ND | ND |
| % Total Impurities | | 0.21 | 1.44 | 2.11 |

**Table 4. Stability Data for Epinephrine Spray Formulation #3 stored at 40°C ± 2°C/ 75 % ± 5 % Relative Humidity**

| 40 °C Formulation #3 | RRT | 0 Week | 2 Weeks | 4 Weeks | 8 Weeks | 3 Months |
|---|---|---|---|---|---|---|
| Appearance | | Clear | Clear | Clear | Clear | Clear |
| Assay (% of initial conc.) | | 100.00 | 98.22 | 96.87 | 94.56 | 93.27 |
| % Racemization | | 0.60 | 1.01 | 1.48 | 3.67 | 4.23 |
| pH | | 4.50 | 3.37 | 3.14 | 3.01 | - |
| % Impurity F | 0.19 | 0.13 | 1.27 | 2.19 | 3.05 | 3.18 |
| % Synephrine | 1.26 | ND | ND | ND | ND | ND |
| % Epinephrone | 1.36 | ND | ND | 0.01 | 0.01 | 0.03 |
| % Methoxy | 1.88 | 0.07 | 0.07 | 0.07 | 0.07 | 0.07 |
| % Unknown Impurity | 0.21 | ND | 0.08 | 0.07 | 0.08 | 0.24 |
| | 3.11 | ND | ND | 0.02 | 0.02 | 0.04 |
| | 3.26 | ND | ND | 0.02 | 0.02 | 0.04 |
| % Total Impurities | | 0.20 | 1.42 | 2.38 | 3.25 | 3.60 |

**Table 5. Stability Data for Epinephrine Spray Formulation #4 stored at 40°C ± 2°C/ 75 % ± 5 % Relative Humidity**

| 40 °C Formulation #4 | RRT | 0 Week | 1 Week | 2 Weeks | 4 Weeks | 6 Weeks | 8 Weeks | 3 Months | 4 Months |
|---|---|---|---|---|---|---|---|---|---|
| Appearance | | Clear | Clear | Clear | Clear | Clear | Clear | Clear | Clear |
| Assay (% of initial conc.) | | 100.0 | 100.7 | 98.54 | 98.35 | 96.02 | 94.44 | 92.60 | 89.34 |
| % Racemization | | 0.60 | 0.68 | - | - | 3.41 | 3.46 | 5.75 | 9.20 |
| % Impurity F | 0.19 | 0.15 | 0.78 | 1.29 | 2.33 | 3.16 | 3.83 | 4.36 | 4.65 |
| % Synephrine | 1.26 | ND | ND | ND | ND | ND | ND | ND | ND |
| % Epinephrone | 1.36 | ND | ND | ND | 0.01 | 0.01 | 0.02 | 0.01 | 0.02 |
| % Methoxy | 1.88 | 0.08 | 0.08 | 0.08 | 0.08 | 0.08 | 0.08 | 0.08 | 0.09 |
| % Unknown Impurity | 0.21 | ND | ND | 0.05 | 0.08 | 0.07 | 0.09 | 0.22 | 0.23 |
| | 3.11 | ND | ND | ND | 0.02 | 0.02 | 0.02 | 0.02 | 0.05 |
| | 3.26 | ND | ND | ND | 0.02 | 0.02 | 0.02 | 0.02 | 0.03 |
| % Total Impurities | | 0.23 | 0.86 | 1.42 | 2.54 | 3.36 | 4.06 | 4.71 | 5.07 |

**Table 6. Stability Data for Epinephrine Spray Formulation #5 stored at 40°C ± 2°C/ 75 % ± 5 % Relative Humidity**

| 40 °C Formulation #5 | RRT | 0 Week | 2 Weeks | 4 Weeks | 8 Weeks | 3 Months |
|---|---|---|---|---|---|---|
| Appearance | | Clear | Clear | Clear | Clear | Clear |
| Assay (% of initial conc.) | | 100.00 | 98.36 | 96.88 | 95.35 | 93.34 |
| % Racemization | | 0.60 | 0.85 | 1.08 | 2.63 | 4.23 |
| pH | | 4.50 | 3.64 | 3.37 | 3.20 | - |
| % Impurity F | 0.19 | 0.15 | 1.48 | 2.50 | 3.50 | 3.75 |
| % Synephrine | 1.26 | ND | ND | ND | ND | ND |
| % Epinephrone | 1.36 | ND | ND | 0.01 | 0.01 | 0.03 |
| % Methoxy | 1.88 | 0.07 | 0.07 | 0.07 | 0.07 | 0.07 |
| % Unknown Impurity | 0.21 | ND | 0.10 | 0.11 | 0.15 | 0.35 |
| | 0.88 | ND | ND | ND | ND | 0.02 |
| | 3.11 | ND | ND | 0.02 | 0.02 | 0.01 |
| | 3.26 | ND | ND | 0.01 | 0.02 | 0.01 |
| % Total Impurities | | 0.22 | 1.65 | 2.72 | 3.77 | 4.24 |

**Table 7. Stability Data for Epinephrine Spray Formulation #6 stored at 40°C ± 2°C/ 75 % ± 5 % Relative Humidity**

| 40 °C Formulation #6 | RRT | 0 Week | 2 Weeks | 1 Month |
|---|---|---|---|---|
| Appearance | | Clear | Clear | Clear |
| Assay (% of initial conc.) | 0.19 | 100.00 | 97.75 | 95.95 |
| % Impurity F | | 0.15 | 2.38 | 4.04 |
| % Synephrine | 1.26 | ND | ND | ND |
| % Epinephrone | 1.36 | ND | 0.01 | 0.01 |
| % Methoxy | 1.88 | 0.04 | 0.04 | 0.04 |
| % Unknown Impurity | 0.21 | ND | 0.05 | 0.08 |
| % Total Impurities | | 0.23 | 2.55 | 4.25 |

**Table 8. Stability Data for Epinephrine Spray Formulation #7 stored at 40°C ± 2°C/ 75 % ± 5 % Relative Humidity**

| 40 °C Formulation #7 | RRT | 0 Week | 2 Weeks | 4 Weeks |
|---|---|---|---|---|
| Appearance | | Clear | Clear | Clear |
| % Impurity F | 0.19 | 0.32 | 1.85 | 2.75 |
| % Synephrine | 1.26 | ND | ND | ND |
| % Epinephrone | 1.36 | ND | ND | ND |
| % Methoxy | 1.88 | 0.07 | 0.07 | 0.07 |
| % Unknown Impurity | 0.21 | 0.17 | 0.36 | 0.44 |
| % Total Impurities | | 0.56 | 2.28 | 3.26 |

**Table 9. Stability Data for Epinephrine Spray Formulation #8 stored at 40°C ± 2°C/ 75 % ± 5 % Relative Humidity**

| 40 °C Formulation #8 | RRT | 0 Week | 2 Weeks |
|---|---|---|---|
| Appearance | | Clear | Light Brown |
| Assay (% of initial conc.) | | 100.00 | 95.55 |
| % Racemization | | 0.63 | 0.62 |
| % Impurity F | 0.19 | 0.22 | 1.56 |
| % Synephrine | 1.26 | ND | ND |
| % Epinephrone | 1.36 | ND | ND |
| % Methoxy | 1.88 | 0.07 | 0.07 |
| % Unknown Impurity | 0.21 | ND | 0.12 |
| | 0.26 | ND | 0.03 |
| | 0.88 | ND | 0.03 |
| | 3.11 | ND | 0.02 |
| | 3.26 | ND | 0.02 |
| % Total Impurities | | 0.27 | 4.68 |

**Table 10. Stability Data for Epinephrine Spray Formulation #9 stored at 40°C ± 2°C/ 75 % ± 5 % Relative Humidity**

| 40 °C Formulation #9 | RRT | 0 Week | 1 Week | 4 Weeks |
|---|---|---|---|---|
| Appearance | | Clear | Clear | Clear |
| Assay (% of initial conc.) | | 100.00 | 100.76 | 99.73 |
| % Racemization | | 0.60 | 0.68 | - |
| % Impurity F | 0.19 | 0.15 | 0.84 | 1.86 |
| % Synephrine | 1.26 | ND | ND | ND |
| % Epinephrone | 1.36 | ND | ND | ND |
| % Methoxy | 1.88 | 0.07 | 0.07 | 0.07 |
| % Unknown Impurity | 0.21 | ND | ND | 0.06 |
| | 3.11 | ND | 0.04 | 0.09 |
| | 3.26 | ND | 0.04 | 0.08 |
| % Total Impurities | | 0.22 | 1.00 | 2.17 |

**Table 11. Stability Data for Epinephrine Spray Formulation #10 stored at 40°C ± 2°C/ 75 % ± 5 % Relative Humidity**

| 40 °C Formulation #10 | RRT | 0 Week | 1 Week |
|---|---|---|---|
| Appearance | | Clear | Clear |
| Assay (% of initial conc.) | | 100.00 | 100.76 |
| % Racemization | | 0.60 | 0.68 |
| % Impurity F | 0.19 | 0.12 | 2.89 |
| % Synephrine | 1.26 | ND | ND |
| % Epinephrone | 1.36 | ND | ND |
| % Methoxy | 1.88 | 0.07 | 0.07 |
| % Unknown Impurity | 0.21 | ND | 0.02 |
| | 3.11 | ND | 0.04 |
| | 3.26 | ND | 0.04 |
| % Total Impurities | | 0.22 | 3.74 |

**Table 12. Stability Data for Epinephrine Spray Formulation #11 stored at 40°C ± 2°C/ 75 % ± 5 % Relative Humidity**

| 40 °C Formulation #11 | RRT | 0 Week | 1 Week |
|---|---|---|---|
| Appearance | | Clear | Clear |
| Assay (% of initial conc.) | | 100.00 | 100.76 |
| % Racemization | | 0.60 | 0.68 |
| % Impurity F | 0.19 | 0.12 | 0.90 |
| % Synephrine | 1.26 | ND | ND |
| % Epinephrone | 1.36 | ND | ND |
| % Methoxy | 1.88 | 0.07 | 0.07 |
| % Unknown Impurity | 0.21 | ND | 0.03 |
| | 3.11 | ND | 0.04 |
| | 3.26 | ND | 0.04 |
| % Total Impurities | | 0.22 | 1.00 |

**Table 13. Stability Data for Epinephrine Spray Formulation #1 stored at 25°C ± 2°C/ 60 % ± 5 % Relative Humidity**

| 25 °C Formulation #1 | RRT | 0 Week | 1 Month | 3 Months |
|---|---|---|---|---|
| Appearance | | Clear | Clear | Clear |
| Assay (% of initial conc.) | | 100.00 | 97.91 | 97.32 |
| % Racemization | | 0.60 | 0.76 | 0.93 |
| % Impurity F | 0.19 | 0.13 | 0.53 | 1.04 |
| % Synephrine | 1.26 | ND | ND | ND |
| % Epinephrone | 1.36 | ND | ND | ND |
| % Methoxy | 1.88 | 0.07 | 0.07 | 0.07 |
| % Unknown Impurity | 0.21 | ND | 0.05 | 0.19 |
| | 3.11 | ND | ND | 0.04 |
| | 3.26 | ND | ND | 0.03 |
| % Total Impurities | | 0.20 | 0.65 | 1.37 |

**Table 14. Stability Data for Epinephrine Spray Formulation #2 stored at 25°C ± 2°C/ 60 % ± 5 % Relative Humidity**

| 25 °C Formulation #2 | RRT | 0 Week | 1 Month | 3 Months |
|---|---|---|---|---|
| Appearance | | Clear | Clear | Clear |
| Assay (% of initial conc.) | | 100.00 | 97.56 | 96.88 |
| % Racemization | | 0.60 | 0.78 | 1.01 |
| % Impurity F | 0.19 | 0.14 | 0.55 | 1.25 |
| % Synephrine | 1.26 | ND | ND | ND |
| % Epinephrone | 1.36 | ND | ND | ND |
| % Methoxy | 1.88 | 0.07 | 0.07 | 0.07 |
| % Unknown Impurity | 0.21 | ND | 0.08 | 0.18 |
| | 3.11 | ND | ND | 0.02 |
| | 3.26 | ND | ND | 0.03 |
| % Total Impurities | | 0.21 | 0.70 | 1.55 |

**Table 15. Stability Data for Epinephrine Spray Formulation #3 stored at 25°C ± 2°C/ 60 % ± 5 % Relative Humidity**

| 25 °C Formulation #3 | RRT | 0 Week | 1 Month | 3 Months |
|---|---|---|---|---|
| Appearance | | Clear | Clear | Clear |
| Assay (% of initial conc.) | | 100.00 | 99.32 | 98.16 |
| % Racemization | | 0.60 | 0.75 | 0.84 |
| % Impurity F | 0.19 | 0.13 | 0.52 | 1.29 |
| % Synephrine | 1.26 | ND | ND | ND |
| % Epinephrone | 1.36 | ND | ND | ND |
| % Methoxy | 1.88 | 0.07 | 0.07 | 0.07 |
| % Unknown Impurity | 0.21 | ND | 0.03 | 0.09 |
| | 3.11 | ND | ND | 0.02 |
| | 3.26 | ND | ND | 0.03 |
| % Total Impurities | | 0.20 | 0.62 | 1.50 |

**Table 16. Stability Data for Epinephrine Spray Formulation #4 stored at 25°C ± 2°C/ 60 % ± 5 % Relative Humidity**

| 25 °C Formulation #4 | RRT | 0 Week | 3 Weeks | 4 Weeks | 8 Weeks | 3 Months | 6 Months |
|---|---|---|---|---|---|---|---|
| Appearance | | Clear | Clear | Clear | Clear | Clear | Clear |
| Assay (% of initial conc.) | | 100.00 | 100.76 | 99.79 | 99.31 | 99.09 | 98.69 |
| % Racemization | | 0.60 | 0.68 | - | 0.91 | 0.97 | - |
| % Impurity F | 0.19 | 0.15 | 0.41 | 0.50 | 1.01 | 1.46 | 2.39 |
| % Synephrine | 1.26 | ND | ND | ND | ND | ND | ND |
| % Epinephrone | 1.36 | ND | ND | ND | ND | 0.01 | 0.01 |
| % Methoxy | 1.88 | 0.08 | 0.08 | 0.08 | 0.08 | 0.08 | 0.08 |
| % Unknown Impurity | 0.21 | ND | 0.05 | 0.06 | 0.06 | 0.11 | 0.10 |
| | 3.02 | ND | ND | ND | ND | ND | 0.12 |
| | 3.11 | ND | ND | ND | ND | ND | 0.12 |
| | 3.26 | ND | ND | ND | ND | ND | 0.10 |
| % Total Impurities | | 0.23 | 0.54 | 0.64 | 1.15 | 1.66 | 2.92 |

**Table 17. Stability Data for Epinephrine Spray Formulation #5 stored at 25°C ± 2°C/ 60 % ± 5 % Relative Humidity**

| 25 °C Formulation #5 | RRT | 0 Week | 1 Month | 3 Months |
|---|---|---|---|---|
| Appearance | | Clear | Clear | Clear |
| Assay (% of initial conc.) | | 100.00 | 98.40 | 97.29 |
| % Racemization | | 0.60 | 0.77 | 0.84 |
| % Impurity F | 0.19 | 0.15 | 0.67 | 1.41 |
| % Synephrine | 1.26 | ND | ND | ND |
| % Epinephrone | 1.36 | ND | ND | ND |
| % Methoxy | 1.88 | 0.07 | 0.07 | 0.07 |
| % Unknown Impurity | 0.21 | ND | 0.05 | 0.18 |
| | 3.11 | ND | ND | 0.01 |
| | 3.26 | ND | ND | 0.01 |
| % Total Impurities | | 0.22 | 0.79 | 1.68 |

**Table 18. Stability Data for Epinephrine Spray Formulation #6 stored at 25°C ± 2°C/ 60 % ± 5 % Relative Humidity**

| 25 °C Formulation #6 | RRT | 0 Week | 2 Weeks | 1 Month | 6 Months |
|---|---|---|---|---|---|
| Appearance | | Clear | Clear | Clear | Clear |
| Assay (% of initial conc.) | | 100.00 | 100.25 | 99.75 | 94.80 |
| % Impurity F | 0.19 | 0.15 | 0.45 | 0.76 | 2.93 |
| % Synephrine | 1.26 | ND | ND | ND | ND |
| % Epinephrone | 1.36 | ND | 0.01 | 0.02 | 0.04 |
| % Methoxy | 1.88 | 0.04 | 0.04 | 0.04 | 0.04 |
| % Unknown Impurity | 0.21 | ND | ND | 0.04 | 0.30 |
| | 0.26 | ND | ND | ND | 0.13 |
| | 0.88 | ND | ND | ND | 0.15 |
| | 2.58 | ND | ND | ND | 0.10 |
| | 3.11 | ND | ND | ND | 0.24 |
| | 3.26 | ND | ND | ND | 0.17 |
| % Total Impurities | | 0.18 | 0.53 | 0.89 | 4.10 |

**Table 19. Stability Data for Epinephrine Spray Formulation #8 stored at 25°C ± 2°C/ 60 % ± 5 % Relative Humidity**

| 25 °C Formulation #8 | RRT | 0 Week | 1 Month |
|---|---|---|---|
| Appearance | | Clear | Clear |
| Assay (% of initial conc.) | | 100.00 | 99.21 |
| % Racemization | | 0.63 | 0.66 |
| % Impurity F | 0.19 | 0.22 | 0.64 |
| % Synephrine | 1.26 | ND | ND |
| % Epinephrone | 1.36 | ND | ND |
| % Methoxy | 1.88 | 0.07 | 0.07 |
| % Unknown Impurity | 0.21 | ND | 0.06 |
| | 3.11 | ND | 0.02 |
| | 3.26 | ND | 0.02 |
| % Total Impurities | | 0.29 | 0.81 |

**Table 20. Stability Data for Epinephrine Spray Formulation #9 stored at 25°C ± 2°C/ 60 % ± 5 % Relative Humidity**

| 25 °C Formulation #9 | RRT | 0 Week | 1 Month | 6 Months |
|---|---|---|---|---|
| Appearance | | Clear | Clear | Clear |
| Assay (% of initial conc.) | | 100.00 | 99.64 | 98.88 |
| % Impurity F | 0.19 | 0.15 | 0.52 | 1.71 |
| % Synephrine | 1.26 | ND | ND | ND |
| % Epinephrone | 1.36 | ND | ND | ND |
| % Methoxy | 1.88 | 0.08 | 0.07 | 0.07 |
| % Unknown Impurity | 0.21 | ND | 0.05 | 0.07 |
| | 3.11 | ND | 0.03 | 0.09 |
| | 3.26 | ND | 0.02 | 0.08 |
| % Total Impurities | | 0.23 | 0.70 | 2.02 |

Formulations #1-#5 had less than 3% total impurities after 4 Weeks (1 Month) at 40°C ± 2°C/75 % ± 5 % Relative Humidity and less than 1% total impurities after 4 Weeks (1 Month) at 25°C ± 2°C/60 % ± 5 % relative humidity. Of Formulations #6-#8, only Formulation #8 was analyzed at 4 Weeks or later at 40°C where 4.68 % total impurities were found. Formulation #9 exhibited total impurities of 2.17 % at 4 weeks 40 °C. Formulation #11 exhibited total impurities of 1 % at one week 40 °C. The superior and surprising stability characteristics of the formulations of the present invention will allow the formulations to be effective when used by patients.

When compared with formulation 4, formulation 7 showed a faster generation of impurities and was not stable for more than a month at 40°C. Formulation 4 was stable for 4 months when stored at 40°C which indicates that EDTA increases the stability of epinephrine formulations.

### Reference Example 3

Formulation #4 was further tested for stability during freeze-thaw cycling. Specifically, Formulation #4 was run through 3 cycles of -20 °C for 48 hours and then 25 °C for 48 hours, where the physical appearance of the formulation was recorded. The formulation remained clear and colorless throughout the entire freeze-thaw cycling indicating a stable formulation throughout.

### Reference Example 4

In order to determine the spray profile of Formulation #4, it was subjected to standardized droplet testing. A challenge of creating an epinephrine spray formulation is that it must be capable of producing spray droplets that are over 10 microns in diameter. Spray droplets of 10 microns or smaller could be inhaled into the lungs.

Droplet analysis was conducted using standard laser analysis procedures known by those of skill in the art. Droplet size distribution (Dv₁₀, Dv₅₀, Dv₉₀, and Span) was tested at two distances, 3 cm and 6 cm. Dv₁₀ refers to the droplet size at which 10% of the volume is smaller; Dv₅₀ refers to the median droplet size; Dv₉₀ refers to droplet size for which 90 % of the total volume is smaller; Span refers to distribution span (Dv90-Dv10)/Dv50. % < 10µm refers to the percentage of the total volume that is made up of droplets less than 10 µm in diameter.

Spray pattern, specifically Dmin, Dmax, and ovality ratio were tested at two distances, 3 cm and 6 cm. Dmin refers to the shortest diameter of the spray pattern in mm, Dmax refers to the widest diameter of the spray pattern in mm, and ovality ratio refers to the ratio of Dmax to Dmin. The spay pattern is measured by shining a laser sheet perpendicular to the spray at a specific distance from the orifice. The ovality ratio is useful as it provides information regarding the shape and density of the spray pump plume.

The results of these tests can be seen below in Tables 17 to 22.

**Table 21. Droplet size distribution of Epinephrine Spray Formulation at 3 cm**

| Droplet Size Distribution 3 cm | DV₁₀ (µm) | DV₅₀ (µm) | DV₉₀ (µm) | % < 10µm | Span |
|---|---|---|---|---|---|
| Min | 15.8 | 30.84 | 124 | 0.05 | 3.145 |
| Max | 17.23 | 33.96 | 228.7 | 0.89 | 6.283 |
| Mean | 16.34 | 32.88 | 177.9 | 0.473 | 4.93 |

**Table 22. Droplet size distribution of Epinephrine Spray Formulation at 6 cm**

| Droplet Size Distribution 6 cm | DV₁₀ (µm) | DV₅₀ (µm) | DV₉₀ (µm) | % < 10µm | Span |
|---|---|---|---|---|---|
| Min | 22.72 | 36.07 | 59.52 | 0 | 1.017 |
| Max | 24.05 | 40.35 | 230.9 | 0 | 5.127 |
| Mean | 23.2 | 38.48 | 121.5 | 0 | 2.49 |

**Table 23. Spray pattern of Epinephrine Spray Formulation at 3 cm**

| Spray Pattern 3 cm | Dmin (mm) | Dmax (mm) | Ovality ratio |
|---|---|---|---|
| Min | 18.9 | 29.8 | 1.415 |
| Max | 22.2 | 32.1 | 1.696 |
| Mean | 20.4 | 31.1 | 1.53 |

**Table 24. Spray pattern of Epinephrine Spray Formulation at 6 cm**

| Spray Pattern 6 cm | Dmin (mm) | Dmax (mm) | Ovality ratio |
|---|---|---|---|
| Min | 26.5 | 47 | 1.68 |
| Max | 32.2 | 54.2 | 1.852 |
| Mean | 29.1 | 51.4 | 1.768 |

**Table 25. Plume geometry of Epinephrine Spray Formulation at 3 cm**

| Plume Geometry 3 cm | Angle (°) | Width (mm) |
|---|---|---|
| Min | 49.2 | 27.6 |
| Max | 63.4 | 37.8 |
| Mean | 55.4 | 32.2 |

**Table 26. Plume geometry of Epinephrine Spray Formulation at 6 cm**

| Plume Geometry 6 cm | Angle (°) | Width (mm) |
|---|---|---|
| Min | 37.7 | 37.7 |
| Max | 43.5 | 43.5 |
| Mean | 40.7 | 40.7 |

Applicant found during testing that formulations of the present invention yielded desirable droplet sizes for spray administration. The testing also revealed that the formulation dose remains consistent when administered with a spray pump.

### Example 5. Preparation of Additional Hydro-alcoholic Epinephrine Spray Formulations.

Additional epinephrine spray formulations were prepared using the components and amounts listed in Table 27 below. All solvents were purged with nitrogen prior to use. Excipients including 0.5 N hydrochloric acid ("HCl"), ethanol, propylene glycol, EDTA, sodium bisulfite, caprylic acid were dissolved in water while stirring at room temperature. Epinephrine base was then added to the excipient solution. Finally, sodium hydroxide ("NaOH")/ hydrochloric acid ("HCl") was used to adjust final pH.

**Table 27 - Additional Hydro-alcoholic Epinephrine Spray Formulations**

| % w/w | #12* | #13* | #14* | #15* | #16* | #17 | #18 | #19 | #20 |
|---|---|---|---|---|---|---|---|---|---|
| Epinephrine base | 3.2 | 3.2 | 3.2 | 3.2 | 3.2 | 3.38 | 3.38 | 3.24 | 3.12 |
| Hydrochloric acid (0.5 N) | 34.6 | 34.6 | 34.6 | 34.6 | 34.6 | 37.7 | 37.7 | 36.2 | 34.8 |
| EDTA | 0.05 | 0.05 | 0.05 | 0.05 | 0.05 | 0.05 | 0.05 | 0.05 | 0.05 |
| Sodium bisulfite | 0.1 | 0.15 | 0.20 | 0.25 | 0.30 | 0.15 | 0.15 | 0.15 | 0.15 |
| Propylene Glycol | 5.0 | 5.0 | 5.0 | 5.0 | 5.0 | 5.0 | 5.0 | 5.0 | 5.0 |
| Caprylic acid | - | - | - | - | - | 2.0 | 10.0 | - | - |
| Benzalkonium chloride | - | - | - | - | - | 0.02 | 0.02 | 0.01 | 0.01 |
| Menthol | 1.0 | 1.0 | 1.0 | 1.0 | 1.0 | 0.5 | 0.5 | - | - |
| Sucralose | 0.5 | 0.5 | 0.5 | 0.5 | 0.5 | 0.5 | 0.5 | - | - |
| Ethanol | 40.0 | 40.0 | 40.0 | 40.0 | 40.0 | 40.0 | 40.0 | 40.0 | 20.0 |
| Water | 15.55 | 15.50 | 15.45 | 15.40 | 15.35 | 10.7 | 2.7 | 15.35 | 36.87 |
| pH adjusted with 2 NaOH/0.5N HCl | 4.5 | 4.5 | 4.5 | 4.5 | 4.5 | 4.5 | 4.5 | 4.5 | 4.5 |

| | | | | | | | | | |
|---|---|---|---|---|---|---|---|---|---|
| * Examples outside the literal scope of the claims due to absence of benzalkonium chloride | | | | | | | | | |

**Table 28 - Additional Aqueous based Epinephrine Spray Formulation**

| % w/w | #21 | #22 | #23 | #24* | #25* | #26* |
|---|---|---|---|---|---|---|
| Epinephrine base | 3.040 | 3.040 | 3.040 | 3.040 | 3.040 | 3.040 |
| Hydrochloric acid (0.5 N) | 33.80 | 33.80 | 33.80 | 33.80 | 33.80 | 33.80 |
| EDTA | 0.05 | 0.05 | 0.05 | 0.05 | 0.05 | 0.05 |
| Sodium bisulfite | 0.150 | 0.250 | 0.5 | 1.00 | 2.00 | 5.00 |
| Benzalkonium chloride | 0.01 | 0.01 | 0.01 | 0.01 | 0.01 | 0.01 |
| Sodium Chloride | 0.6 | 0.6 | 0.6 | 0.6 | 0.6 | 0.6 |
| Water | 62.35 | 62.25 | 62.00 | 61.5 | 60.5 | 57.5 |
| pH adjusted with 2 NaOH/0.5N HCl | 4.5 | 4.5 | 4.5 | 4.5 | 4.5 | 4.5 |

| | | | | | | |
|---|---|---|---|---|---|---|
| *Examples outside the literal scope of the claims due to excess sodium bisulfite | | | | | | |

The formulations listed in Table 27 and Table 28 were filled in unit dose devices, packed into an oxygen impermeable pouches with oxygen scavengers, and then subjected to stability at 40°C ± 2°C/75 % ± 5 % relative humidity and 25°C ± 2°C/60 % ± 5 % relative humidity. The stability of the formulations was analyzed at specified time points by evaluating their potency (assay value) and impurity levels. Assay and impurities were detected using high-performance liquid chromatography with an ultraviolet detector. The assay was performed at 280 nm and indicated as a % of initial concentration. For all impurities, analysis was performed at 210 nm and 280nm and expressed as a % area. Amounts of particular impurities are listed in Tables 29 to 40 as a percentage of area of each formulation along with amount of total impurities. Relative retention time ("RRT") is given for each impurity. "ND" indicates that the impurity was not detected.

**Table 29. Stability Data for Epinephrine Spray Formulations # 12* to # 16* stored at 25°C ± 2°C/ 60 % ± 5 % Relative Humidity**

| | | Stability @18M 25°C/ 60%RH | | | | | |
|---|---|---|---|---|---|---|---|
| | RRT | 0 Week | F # 12 18 months | F # 13 18 Months | F # 14 18 Months | F # 15 18 months | F # 16 18 months |
| Appearance | | Clear | Clear | Clear | Clear | Clear | Clear |
| % Impurity F | 0.19 | 0.11 | 1.91 | 2.53 | 3.27 | 3.57 | 4.79* |
| % Synephrine | 1.26 | ND | ND | ND | ND | ND | ND |
| % Epinephrone | 1.36 | ND | 0.01 | 0.02 | 0.01 | 0.01 | 0.01 |
| %Methoxy | 1.90 | 0.07 | 0.08 | 0.08 | 0.07 | 0.07 | 0.08 |
| % Unknown Impurities | 0.22 | 0.01 | 0.12 | 0.12 | 0.13 | 0.12 | 0.12 |
| | 0.25 | ND | 0.09 | 0.03 | 0.03 | ND | 0.07 |
| | 0.26 | ND | ND | ND | ND | ND | ND |
| | 0.64 | ND | ND | ND | ND | ND | 0.04 |
| | 0.88 | ND | 0.03 | 0.03 | 0.04 | 0.02 | 0.01 |
| | 1.21 | ND | 0.09 | 0.09 | 0.08 | 0.04 | 0.06 |
| | 1.34 | ND | 0.02 | 0.05 | 0.03 | 0.04 | 0.03 |
| | 1.50 | ND | ND | ND | 0.07 | 0.04 | 0.03 |
| | 1.51 | ND | 0.1 | 0.07 | ND | ND | ND |
| | 2.43 | ND | ND | ND | ND | 0.06 | ND |
| | 2.44 | ND | ND | 0.07 | 0.08 | ND | ND |
| | 2.45 | ND | 0.11 | ND | ND | ND | ND |
| | 2.57 | ND | ND | ND | ND | ND | 0.06 |
| | 2.71 | ND | ND | ND | ND | ND | ND |
| | 2.90 | ND | ND | ND | 0.03 | ND | ND |
| | 2.91 | ND | ND | 0.04 | ND | ND | ND |
| | 2.93 | ND | 0.07 | ND | ND | ND | ND |
| | 2.96 | ND | ND | ND | ND | ND | ND |
| | 3.11 | ND | 0.05 | 0.03 | 0.03 | 0.03 | 0.02 |
| | 3.26 | ND | ND | 0.04 | ND | ND | ND |
| Total Impurities | | 0.19 | 2.68 | 3.20 | 3.87 | 4.00 | 5.32 |

**Table 30. Stability Data for Epinephrine Spray Formulation # 17 stored at 25°C ± 2°C/ 60 % ± 5 % Relative Humidity**

| | RRT | 1 Month | 2 Month |
|---|---|---|---|
| Appearance | | Clear | Clear |
| % Impurity F | 0.19 | 0.50 | 0.72 |
| % Epinephrone | 1.36 | ND | ND |
| % Methoxy | 1.90 | 0.07 | 0.07 |
| % Unknown Impurities | 1.08 | 0.14 | 0.08 |
| % Total Impurities | | 0.71 | 0.87 |

**Table 31. Stability Data for Epinephrine Spray Formulation # 18 stored at 25°C ± 2°C/ 60 % ± 5 % Relative Humidity**

| | RRT | 1 Month | 2 Months |
|---|---|---|---|
| Appearance | Clear | Clear | Clear |
| % Impurity F | 0.19 | 0.57 | 0.75 |
| % Epinephrone | 1.36 | ND | ND |
| % Methoxy | 1.90 | 0.07 | 0.07 |
| | 1.08 | 0.08 | 0.14 |
| | 1.12 | BQL | 0.07 |
| % Total Impurities | | 0.72 | 1.03 |

**Table 32. Stability Data for Epinephrine Spray Formulations # 19 stored at 25°C ± 2°C/ 60 % ± 5 % Relative Humidity**

| | RRT | 0 Week | 1 Month | 3 Months |
|---|---|---|---|---|
| Assay | - | 101.32 | 99.22 | 100.49 |
| Appearance | - | Clear | Clear | Clear |
| % Impurity F | 0.19 | 0.16 | 0.47 | 1.03 |
| % Epinephrone | 1.34 | ND | 0.01 | 0.01 |
| % Methoxy | 1.77 | 0.07 | 0.06 | 0.06 |
| % Unknown Impurities | 0.21 | ND | ND | 0.06 |
| | 0.71 | ND | 0.02 | 0.01 |
| | 0.88 | ND | 0.01 | 0.01 |
| | 1.23 | ND | ND | 0.02 |
| | 1.42 | ND | ND | 0.01 |
| | 1.53 | ND | 0.01 | ND |
| | 1.60 | ND | ND | 0.01 |
| | 1.64 | ND | ND | 0.01 |
| | 1.86 | ND | 0.01 | ND |
| | 2.55 | ND | 0.02 | 0.02 |
| | 2.56 | ND | 0.02 | ND |
| | 2.86 | ND | 0.02 | 0.01 |
| | 2.93 | ND | 0.01 | 0.02 |
| | 3.05 | 0.01 | 0.03 | ND |
| | 3.22 | 0.01 | 0.03 | 0.03 |
| | 3.44 | 0.01 | 0.03 | 0.02 |
| Total impurities (%) | | 0.26 | 0.75 | 1.33 |

**Table 33. Stability Data for Epinephrine Spray Formulations # 20 stored at 25°C ± 2°C/ 60 % ± 5 % Relative Humidity**

| | RRT | 0 Week | 1 Month | 3 Months |
|---|---|---|---|---|
| Assay (% LC) | - | 103.9307 | 102.23 | 99.07 |
| Appearance | | Clear | Clear | Clear |
| % Impurity F | 0.19 | 0.16 | 0.53 | 1.25 |
| % Epinephrone | 1.35 | ND | 0.01 | 0.01 |
| % Methoxy | 1.78 | 0.06 | 0.06 | 0.06 |
| % Unknown Impurities | 0.21 | ND | ND | 0.05 |
| | 0.70 | ND | 0.03 | 0.02 |
| | 0.83 | ND | 0.01 | 0.01 |
| | 0.88 | 0.01 | 0.01 | 0.01 |
| | 1.38 | ND | ND | 0.01 |
| | 1.51 | 0.04 | ND | ND |
| | 1.55 | ND | 0.01 | 0.01 |
| | 2.55 | ND | 0.01 | ND |
| | 2.56 | ND | 0.01 | 0.01 |
| | 2.61 | ND | 0.02 | ND |
| | 2.75 | ND | ND | 0.01 |
| | 2.80 | ND | ND | 0.02 |
| | 2.87 | ND | 0.02 | ND |
| | 2.96 | ND | 0.01 | ND |
| | 3.09 | 0.02 | 0.02 | ND |
| | 3.22 | ND | ND | 0.01 |
| | 3.45 | ND | ND | 0.02 |
| Total impurities (%) | | 0.22 | 0.75 | 1.50 |

**Table 34. Stability Data for Epinephrine Spray Formulations # 12* to 16* stored at 40 °C ± 2°C/ 75 % ± 5 % Relative Humidity**

| Epinephrine | | | F# 12 | F# 13 | F# 14 | F# 15 | F# 16 |
|---|---|---|---|---|---|---|---|
| | RRT | 0 Week | 2 Months | 2 Months | 2 Months | 2 Months | 2 Months |
| Assay | | 100.00 | 98.13 | 94.51 | 93.02 | 93.26 | 92.02 |
| Appearance | | Clear | Clear | Clear | Clear | Clear | Clear |
| % Impurity F | 0.19 | 0.11 | 3.22 | 5.64 | 7.42 | 9.7 | 11.3 |
| % Synephrine | 1.26 | ND | ND | ND | ND | ND | ND |
| % Epinephrone | 1.36 | ND | 0.01 | 0.01 | 0.01 | 0.02 | 0.02 |
| % Methoxy | 1.90 | 0.07 | 0.06 | 0.06 | 0.06 | 0.06 | 0.06 |
| % Unknown Impurities | 0.22 | 0.01 | 0.16 | 0.16 | 0.15 | 0.14 | 0.12 |
| | 0.25 | ND | 0.06 | 0.06 | 0.05 | 0.05 | 0.05 |
| | 0.26 | ND | ND | 0.01 | 0.01 | 0.01 | 0.01 |
| | 0.88 | ND | ND | 0.01 | 0.01 | 0.02 | 0.01 |
| | 1.21 | ND | 0.06 | 0.06 | 0.05 | 0.04 | 0.03 |
| | 1.34 | ND | 0.02 | 0.02 | 0.02 | 0.02 | 0.01 |
| | 1.55 | ND | 0.03 | 0.03 | 0.02 | 0.02 | 0.02 |
| | 2.57 | ND | 0.18 | 0.11 | 0.1 | 0.08 | 0.07 |
| | 3.11 | ND | 0.03 | 0.02 | 0.01 | 0.01 | 0.01 |
| | 3.26 | ND | 0.02 | 0.01 | 0.01 | 0.02 | ND |
| % Total Impurities | | 0.19 | 3.85 | 6.20 | 7.92 | 10.19 | 11.71 |

**Table 35. Stability Data for Epinephrine Spray Formulations # 17 stored at 40 °C ± 2°C/ 75 % ± 5 % Relative Humidity**

| | RRT | 2 Weeks | 4 Weeks | 6 Weeks | 3 Months |
|---|---|---|---|---|---|
| Physical appearance | | Clear | Clear | Clear | Clear |
| % Impurity F | 0.19 | 1.02 | 2.03 | 3.01 | 5.11 |
| % Epinephrone | 1.36 | ND | ND | BQL | BQL |
| % Methoxy | 1.90 | 0.07 | 0.07 | 0.07 | 0.06 |
| | 1.08 | 0.08 | 0.15 | 0.15 | 0.09 |
| | 1.12 | ND | ND | 0.15 | 0.15 |
| | 1.21 | ND | BQL | 0.06 | 0.13 |
| | 2.53 | BQL | BQL | 0.05 | 0.15 |
| | 2.58 | ND | ND | BQL | 0.06 |
| | 3.04 | ND | ND | BQL | 0.06 |
| % Total Impurities | | 1.17 | 2.25 | 3.49 | 5.81 |

**Table 36. Stability Data for Epinephrine Spray Formulations #18 stored at 40 °C ± 2°C/ 75 % ± 5 % Relative Humidity**

| | RRT | 2 Weeks | 1 Month | 2 Months |
|---|---|---|---|---|
| Appearance | | Clear | Clear | Clear |
| % Impurity F | 0.19 | 0.93 | 2.16 | 3.71 |
| % Epinephrone | 1.36 | ND | 0.01 | BQL |
| % Methoxy | 1.90 | 0.07 | 0.07 | 0.07 |
| | 1.08 | 0.09 | 0.09 | 0.10 |
| | 1.12 | BQL | 0.06 | 0.14 |
| | 1.21 | ND | BQL | 0.18 |
| | 2.53 | 0.09 | 0.23 | 0.17 |
| | 2.58 | BQL | 0.08 | 0.07 |
| | 3.04 | BQL | BQL | 0.05 |
| % Total Impurities | | 1.18 | 2.7 | 4.49 |

**Table 37. Stability Data for Epinephrine Spray Formulations #19 stored at 40 °C ± 2°C/ 75 % ± 5 % Relative Humidity**

| | RRT | 0 Week | 1 Months | 2 Months | 3 Months | 4 Months |
|---|---|---|---|---|---|---|
| Assay | | 101.32 | 101.11 | 97.06 | 95.95 | 96.08 |
| Appearance | | Clear | Clear | Clear | Clear, Light Yellow | Clear, Light Yellow |
| % Impurity F | 0.19 | 0.16 | 1.92 | 4.25 | 6.42 | 7.20 |
| % Epinephrone | 1.34 | ND | 0.01 | 0.01 | 0.01 | 0.01 |
| % Methoxy | 1.77 | 0.07 | 0.06 | 0.06 | 0.06 | 0.06 |
| % Unknown Impurities | 0.21 | ND | 0.09 | 0.19 | 0.11 | 0.3 |
| | 0.26 | ND | ND | 0.14 | 0.21 | 0.31 |
| | 0.52 | ND | ND | ND | ND | 0.03 |
| | 0.64 | ND | ND | ND | ND | 0.02 |
| | 0.71 | ND | ND | ND | 0.01 | ND |
| | 0.84 | ND | ND | ND | ND | 0.01 |
| | 0.88 | ND | 0.01 | ND | ND | 0.01 |
| | 1.17 | ND | 0.06 | 0.07 | 0.05 | 0.09 |
| | 1.28 | ND | 0.02 | 0.04 | 0.02 | 0.02 |
| | 1.38 | ND | 0.02 | 0.04 | 0.02 | 0.02 |
| | 1.55 | ND | ND | ND | 0.02 | ND |
| | 1.68 | ND | ND | ND | 0.01 | 0.02 |
| | 1.83 | ND | ND | ND | 0.01 | ND |
| | 1.99 | ND | ND | ND | ND | 0.01 |
| | 2.07 | ND | ND | ND | ND | 0.01 |
| | 2.38 | ND | 0.04 | ND | ND | ND |
| | 2.57 | ND | 0.01 | ND | ND | ND |
| | 2.60 | ND | 0.02 | 0.05 | 0.1 | 0.19 |
| | 2.80 | ND | ND | ND | 0.02 | ND |
| | 2.86 | ND | 0.02 | ND | ND | ND |
| | 2.98 | ND | 0.01 | ND | ND | ND |
| | 3.05 | 0.01 | ND | ND | ND | ND |
| | 3.13 | ND | 0.03 | ND | ND | ND |
| | 3.22 | ND | ND | ND | 0.03 | ND |
| | 3.26 | ND | 0.03 | ND | 0.03 | ND |
| | 3.33 | ND | ND | ND | ND | 0.07 |
| | 3.38 | ND | ND | ND | ND | 0.02 |
| | 3.44 | ND | ND | ND | 0.02 | 0.14 |
| | 3.68 | ND | ND | ND | 0.02 | 0.03 |
| Total impurities (%) | | 0.24 | 2.33 | 4.85 | 7.15 | 8.57 |

**Table 38 Stability Data for Epinephrine Spray Formulations # 20 stored at 40 °C ± 2°C/ 75 % ± 5 % Relative Humidity**

| | RRT | 0 Week | 1 Month | 2 Months | 3 Months | 4 Months |
|---|---|---|---|---|---|---|
| Assay | - | 103.93 | 99.44 | 96.16 | 96.35 | 96.38 |
| Appearance | | Clear | Clear | Clear | Clear | Clear |
| % Impurity F | 0.19 | 0.16 | 2.61 | 5.66 | 7.69 | 6.30 |
| % Epinephrone | 1.35 | ND | 0.01 | 0.01 | 0.01 | 0.01 |
| % Methoxy | 1.78 | 0.06 | 0.07 | 0.06 | 0.06 | 0.06 |
| Unknown Impurities | 0.21 | ND | 0.08 | 0.10 | 0.09 | 0.29 |
| | 0.26 | ND | ND | 0.06 | 0.12 | 0.17 |
| | 0.70 | ND | 0.02 | 0.02 | 0.02 | 0.02 |
| | 0.88 | 0.01 | 0.01 | ND | 0.01 | 0.01 |
| | 1.18 | ND | 0.03 | 0.04 | 0.03 | 0.11 |
| | 1.34 | ND | ND | ND | ND | 0.04 |
| | 1.36 | ND | ND | ND | 0.02 | 0.03 |
| | 1.49 | ND | 0.01 | ND | ND | ND |
| | 1.42 | ND | ND | ND | 0.02 | ND |
| | 1.51 | 0.04 | ND | ND | ND | ND |
| | 1.55 | ND | 0.01 | 0.02 | 0.02 | ND |
| | 1.58 | ND | ND | ND | ND | 0.09 |
| | 1.64 | ND | ND | ND | 0.01 | ND |
| | 1.89 | ND | ND | ND | 0.01 | ND |
| | 2.07 | ND | ND | ND | ND | 0.02 |
| | 2.39 | ND | 0.02 | ND | ND | 0.01 |
| | 2.56 | ND | 0.01 | ND | ND | ND |
| | 2.61 | ND | 0.02 | 0.04 | 0.07 | ND |
| | 2.71 | ND | ND | ND | ND | 0.39 |
| | 2.78 | ND | ND | ND | ND | 0.20 |
| | 2.87 | ND | 0.01 | ND | 0.02 | 0.01 |
| | 2.96 | ND | 0.02 | ND | ND | ND |
| | 3.09 | 0.02 | 0.01 | ND | ND | ND |
| | 3.14 | ND | 0.02 | ND | ND | ND |
| | 3.22 | ND | ND | ND | 0.14 | 0.16 |
| | 3.36 | ND | ND | ND | 0.03 | ND |
| | 3.49 | ND | ND | ND | ND | 0.22 |
| | 3.55 | ND | ND | ND | 0.02 | 0.02 |
| | 3.60 | ND | ND | 0.05 | ND | ND |
| Total impurities (%) | | 0.22 | 2.96 | 6.06 | 8.39 | 8.16 |

**Table 39. Stability Data for Epinephrine Spray Formulations # 21 stored at 40 °C ± 2°C/ 75 % ± 5 % Relative Humidity**

| | | 0 Week | 1Month | 2Month |
|---|---|---|---|---|
| Assay | | 98.20 | 94.84 | 94.14 |
| Appearance | | Clear | Clear | Clear |
| % Impurity F | 0.19 | 0.16 | 4.43 | 7.96 |
| % Epinephrone | 1.34 | ND | 0.01 | 0.01 |
| % Methoxy | 1.77 | 0.06 | 0.06 | 0.06 |
| % Unknown Impurities | 0.21 | ND | 0.06 | ND |
| | 0.71 | ND | 0.03 | ND |
| | 0.81 | ND | 0.01 | ND |
| | 1.08 | ND | ND | 0.09 |
| | 1.17 | ND | ND | ND |
| | 1.38 | ND | 0.01 | ND |
| | 1.60 | ND | 0.01 | ND |
| | 2.15 | ND | 0.01 | ND |
| | 2.76 | ND | 0.01 | ND |
| | 2.81 | ND | 0.01 | ND |
| | 2.91 | 0.02 | ND | ND |
| | 3.13 | ND | ND | 0.03 |
| | 3.22 | ND | 0.04 | ND |
| | 3.44 | 0.02 | 0.02 | ND |
| Total impurities (%) | | 0.26 | 4.71 | 8.15 |

**Table 40. Stability Data for Epinephrine Spray Formulations # 21 stored at 25°C ± 2°C/ 60 % ± 5 % Relative Humidity**

| | RRT | 0 Week | 1 Month | 2 Months |
|---|---|---|---|---|
| Assay | | 98.20 | 101.02 | 99.07 |
| Appearance | | Clear | Clear | Clear |
| % Impurity F | 0.19 | 0.16 | 0.72 | 1.38 |
| % Epinephrone | 1.34 | ND | 0.01 | 0.01 |
| % Methoxy | 1.77 | 0.06 | 0.06 | 0.06 |
| % Unknown Impurities | 0.21 | ND | 0.03 | 0.04 |
| | 0.71 | ND | 0.04 | 0.03 |
| | 0.81 | ND | 0.03 | 0.02 |
| | 1.38 | ND | ND | 0.01 |
| | 1.60 | ND | 0.01 | ND |
| | 1.68 | ND | ND | ND |
| | 1.83 | ND | ND | ND |
| | 2.15 | ND | 0.02 | ND |
| | 2.38 | ND | ND | ND |
| | 2.76 | ND | 0.01 | ND |
| | 2.81 | ND | 0.02 | ND |
| | 2.86 | ND | ND | ND |
| | 2.91 | 0.02 | ND | ND |
| | 3.22 | ND | 0.01 | ND |
| | 3.44 | 0.02 | 0.02 | ND |
| Total impurities (%) | | 0.26 | 0.98 | 1.55 |

All formulas exemplified in Tables 27 and 28 above, remained clear throughout stability testing. Formulations were filled in to unit dose spray devices under nitrogen blanket, and then subjected to accelerated stability testing at 25 °C ± 2°C/ 60 % ± 5 % and 40 °C ± 2°C/ 75 % ± 5 % without pouching (Formulations 12 to 18), while formulations (F# 19 to 26) were subjected to accelerated stability testing under similar conditions with pouching containing oxygen scavenges. Formulation 13, un-pouched showed impurity F levels at 5.64 % at 2M/40°C. However similar formulation 19, pouched, exhibited impurity F levels at 4.25% at 2M/40°C. Stability data suggest that epinephrine spray formulations which are pouched with oxygen scavenges are more stable compare to un-pouched formulations. Both hydro-alcoholic and aqueous formulations of present invention are stable at room temperature as well as 40°C ± 2°C/ 75 % ± 5.

### Example 6. Mini-pig pharmacokinetic data for epinephrine formulations

Protocol design was a single-dose crossover study. Five healthy male Yucatan mini-pigs weighing approximately eighty to ninety-five kilograms each were administered epinephrine formulations. The minipigs were fasted overnight till four hours' post administration. Each dosing was followed by a washout period of at least one-week. Blood samples were taken prior to administration and 2, 5, 10, 15, 20, 30, 45 min, 1, 1.5, 2, 4, and 24 hours' post dose. Minipig plasma samples were measured for epinephrine concentrations via liquid chromatography-tandem mass spectrometry.

The following pharmacokinetic parameters were calculated: peak concentration in plasma (Cₘₐₓ), time to reach Cₘₐₓ (Tₘₐₓ), and area under the concentration-time curve from time-zero to 24 hours postdose (AUC₀₋₂₄ₕ).

The pharmacokinetic behavior of epinephrine formulations was evaluated. At 5 minutes after a single-dose intramuscular (IM) administration of epinephrine in minipigs, the geometric mean plasma concentration of epinephrine was 0.046 ng/mL. When epinephrine was formulated in #M1 and administered intranasally, a more rapid absorption of epinephrine was observed. Specifically, #M1 showed a geometric mean epinephrine plasma concentration of 0.26 ng/mL at 5 minutes postdose. It was also noted that a plasma concentration of 0.41 ng/mL was achieved as early as 2 minutes after an intranasal administration of #M1. In addition, #M1 showed a geometric mean Cmax of 1.01 ng/mL and a geometric mean AUC₀₋₂₄ₕ of 161.0 ng*min/L.

**Table 41. Epinephrine Formulations for Minipig Dosing**

| Formulation | #M1 |
|---|---|
| Epinephrine base | 3.25 |
| Dilute Hydrochloric acid 0.5 N | 36.2 |
| Benzalkonium Chloride | 0.01 |
| Ethanol | 40 |
| Propylene Glycol | 5 |
| Sodium Bisulfite | 0.15 |
| EDTA | 0.05 |
| Water | 15.34 |
| Total | 100 |

| | |
|---|---|
| Components: %w/w | |

**Table 42. Plasma concentrations for epinephrine after administration to Yucatan minipigs under fasted conditions.**

| Formulation | IM solution | #M1 |
|---|---|---|
| Route of administration | IM | Intranasal |
| Dose per animal (mg) | 0.3 | 6 |
| Concentration @ 2 min (ng/mL) | 0.036 | 0.41 |
| Concentration @ 5 min (ng/mL) | 0.046 | 0.26 |
| Concentration @ 10 min (ng/mL) | 0.073 | 0.31 |
| Concentration @ 15 min (ng/mL) | 0.103 | 0.17 |
| Concentration @ 30 min (ng/mL) | 0.100 | 0.19 |
| Tₘₐₓ (min) | 90 | 5 |
| Cₘₐₓ (ng/mL) | 0.25 | 1.01 |
| AUC₀₋₂₄ₕ (ng*min/mL) | 173.7 | 161.0 |

| | | |
|---|---|---|
| Plasma concentration: geometric mean Tₘₐₓ: median value Cₘₐₓ and AUC₀₋₂₄ₕ: geometric mean | | |

### Reference Example 7. Rabbit pharmacokinetic data for epinephrine formulations

Protocol design was a single-dose non-crossover study. Pharmacokinetics of a number of epinephrine formulations were evaluated in healthy male New Zealand white rabbits weighing approximately two to three kilograms. For dosing of each formulation, five or six rabbits were used. The rabbits were fasted overnight till four hours' post administration. Blood samples were taken prior to administration and 5, 10, 15, 20, 30, 40 min, 1, 1.5, 2, 4, and 5 hours' post dose. Rabbit plasma samples were measured for epinephrine concentrations via liquid chromatography-tandem mass spectrometry.

Formulation #R1-#R8 were provided in Table 43 and 44. Formulation #R9 existed as a spray-dried powder of epinephrine bitartrate salt. For the evaluation of #R9, approximately 21.83 mg of powder was dosed to each animal, as an equivalent dose of 12 mg epinephrine.

The following pharmacokinetic parameters were calculated: peak concentration in plasma (Cₘₐₓ), time to reach Cₘₐₓ (Tₘₐₓ), and area under the concentration-time curve from time-zero to 24 hours postdose (AUC₀₋₂₄ₕ).

The pharmacokinetic behavior of epinephrine formulations was evaluated. At 5 minutes after a single-dose IM administration of epinephrine in rabbits, the mean plasma concentration of epinephrine was 0.988 ng/mL. In comparison, a sublingual administration of #R9 enabled a similar absorption of epinephrine in the early phase. Specifically, #R9 showed a mean epinephrine plasma concentration of 0.0.814 ng/mL at 5 minutes postdose. It was also noted that a plasma concentration of 0.713 ng/mL was achieved at 5 minutes after a sublingual administration of #R6. In addition, #R9 showed a geometric mean Cmax of 7.0 ng/mL and a geometric mean AUC₀₋₂₄ₕ of 1050.1 ng*min/L.

**Table 43. Epinephrine Formulations for Rabbit Dosing**

| Formulation | #R1 | #R2 | #R3 | #R4 | #R5 |
|---|---|---|---|---|---|
| Epinephrine base | 0 | 0.006 | 3.025 | 3.243 | 3.243 |
| Dilute Hydrochloric Acid 0.5 N | 0.3 | 0.5 | 33.27 | 36.2 | 36.2 |
| Dehydrated Alcohol | | | | 40 | 40 |
| Menthol | | | | 1 | 1 |
| Sucralose | 0.5 | | 0.5 | 0.5 | 0.5 |
| Propylene Glycol | | | | 5 | 5 |
| Sodium Bisulfite | 0.2 | 0.01 | 0.2 | 0.3 | 0.3 |
| Edetate Disodium (EDTA) | 0.05 | | 0.05 | 0.05 | 0.05 |
| Water USP | 98.45 | 98.584 | 62.455 | 13.707 | 13.707 |
| Total | 100 | 100 | 100 | 100 | 100 |

| | | | | | |
|---|---|---|---|---|---|
| Components: %w/w | | | | | |

**Table 44. Additional Epinephrine Formulations for Rabbit Dosing**

| Formulation | #R6 | #R7 | #R8 |
|---|---|---|---|
| Epinephrine base | 6.648 | 12.88 | 12.88 |
| Dilute Hydrochloric Acid 1.5 N | 24.6 | | |
| Dilute Hydrochloric Acid 3 N | | 24.2 | 24.2 |
| Dehydrated Alcohol | 40 | 40 | 40 |
| Menthol | 0.5 | 0.5 | 0.5 |
| Sucralose | 0.5 | 0.5 | 0.5 |
| Propylene Glycol | 5 | 5 | 5 |
| Sodium Bisulfite | 0.6 | 0.6 | 0.6 |
| Edetate Disodium (EDTA) | 0.1 | 0.1 | 0.1 |
| Water USP | 21.552 | 15.72 | 15.72 |
| Caprylic acid | 0.5 | | |
| Capric acid | | | 0.5 |
| Total | 100 | 100 | 100 |

| | | | |
|---|---|---|---|
| Components: %w/w | | | |

**Table 45. Plasma concentrations for epinephrine after administration to rabbits under fasted conditions.**

| Formulation | #R1 | #R2 | #R3 | #R4 | #R5 |
|---|---|---|---|---|---|
| Route of administration | SL | IM | SL | SL | SL |
| Dose per animal (mg) | 0 | 0.03 | 3 | 3 | 6 |
| Concentration @ 5 min (ng/mL) | 0.020 | 0.99 | 0.28 | 0.19 | 0.11 |
| Concentration @ 10 min (ng/mL) | 0.011 | 0.83 | 0.24 | 0.32 | 0.35 |
| Concentration @ 15 min (ng/mL) | 0.014 | 0.79 | 0.49 | 0.55 | 0.86 |
| Concentration @ 30 min (ng/mL) | 0.012 | 0.73 | 0.44 | 1.24 | 1.94 |
| Tₘₐₓ (min) | 300 | 15 | 240 | 90 | 90 |
| Cₘₐₓ (ng/mL) | 0.05 | 1.1 | 1.3 | 2.9 | 6.3 |
| AUC₀₋₂₄ₕ (ng*min/mL) | 6.7 | 96.7 | 217.0 | 537.1 | 956.8 |

| | | | | | |
|---|---|---|---|---|---|
| Plasma concentration: mean Tₘₐₓ: median value Cₘₐₓ and AUC₀₋₂₄ₕ: geometric mean IM denotes intramuscular, SL denotes sublingual | | | | | |

**Table 46. Plasma concentrations for epinephrine after administration to rabbits under fasted conditions.**

| Formulation | #R6 | #R7 | #R8 | #R9 |
|---|---|---|---|---|
| Route of administration | SL | SL | SL | SL |
| Dose per animal (mg) | 6 | 12 | 12 | 12 |
| Concentration @ 5 min (ng/mL) | 0.71 | 0.28 | 0.12 | 0.81 |
| Concentration @ 10 min (ng/mL) | 0.37 | 0.46 | 0.17 | 1.10 |
| Concentration @ 15 min (ng/mL) | 0.51 | 0.83 | 0.53 | 1.40 |
| Concentration @ 30 min (ng/mL) | 0.93 | 2.75 | 3.34 | 3.57 |
| Tₘₐₓ (min) | 120 | 120 | 180 | 65 |
| Cₘₐₓ (ng/mL) | 3.1 | 6.0 | 14.3 | 7.0 |
| AUC₀₋₂₄ₕ (ng*min/mL) | 482.2 | 867.0 | 2145.1 | 1050.1 |

| | | | | |
|---|---|---|---|---|
| Plasma concentration: mean Tₘₐₓ: median value Cₘₐₓ and AUC₀₋₂₄ₕ: geometric mean | | | | |

## Claims

1. An epinephrine spray formulation for use in a method of treating anaphylaxis, wherein the formulation comprises:
from about 0.1 % to about 15 % w/w epinephrine, or a salt thereof;
from about 1 % to about 65 % w/w hydrochloric acid with a normality from about 0.1 to 12N;
from about 2 % to about 60 % w/w ethanol;
from about 2% to about 98 % w/w water;
from about 0.001 % to about 0.5 % w/w sodium bisulfite;
from about 0.005 % to about 1 % w/w edetate disodium dihydrate and,
from about 0.001% to about 0.5% w/w benzalkonium chloride; and
wherein the formulation is administered via the intranasal route;
wherein w/w denotes weight by weight of the formulation and, wherein "about" is defined as ± 10 % of the value modified.

2. The epinephrine spray formulation for use according to claim 1, wherein the formulation comprises:
about 3.2 % w/w epinephrine, or a salt thereof;
about 35.5 % w/w hydrochloric acid with a normality of 0.5;
about 40 % w/w ethanol;
about 16.1 % w/w water;
about 0.05 % w/w edetate disodium dihydrate;
about 0.15 % w/w sodium bisulfite;
about 0.01 % w/w benzalkonium chloride.

3. The epinephrine spray formulation for use according to claim 2, wherein the formulation further comprises about 5% w/w propylene glycol.

## Patentansprüche

1. Epinephrinsprühformulierung zur Verwendung in einem Verfahren zum Behandeln von Anaphylaxie, wobei die Formulierung Folgendes umfasst:
von etwa 0,1 Gew.-% bis etwa 15 Gew.-% Ephinephrin oder ein Salz davon;
von etwa 1 Gew.-% bis etwa 65 Gew.-% Salzsäure mit einer Normalität von etwa 0,1 bis 12 N;
von etwa 2 Gew.-% bis etwa 60 Gew.-% Ethanol;
von etwa 2 Gew.-% bis etwa 98 Gew.-% Wasser;
von etwa 0,001 Gew.-% bis etwa 0,5 Gew.-% Natriumbisulfit;
von etwa 0,005 Gew.-% bis etwa 1 Gew.-% Edetat-Dinatriumdihydrat und
von etwa 0,001 Gew.-% bis etwa 0,5 Gew.-% Benzalkoniumchlorid; und
wobei die Formulierung über den intranasalen Weg verabreicht wird;
wobei Gew.-% das Gewichtsprozent der Formulierung bezeichnet und wobei "etwa" als ± 10 % des modifizierten Werts definiert ist.

2. Epinephrinsprühformulierung zur Verwendung nach Anspruch 1, wobei die Formulierung Folgendes umfasst:
etwa 3,2 Gew.-% Ephinephrin oder ein Salz davon;
etwa 35,5 Gew.-%Salzsäure mit einer Normalität von 0,5;
etwa 40 Gew.-% Ethanol;
etwa 16,1 Gew.-% Wasser;
etwa 0,05 Gew.-% Edetat-Dinatriumdihydrat;
etwa 0,15 Gew.-% Natriumbisulfit;
etwa 0,01 Gew.-% Benzalkoniumchlorid.

3. Epinephrinsprühformulierung zur Verwendung nach Anspruch 2, wobei die Formulierung ferner etwa 5 Gew.-% Propylenglykol umfasst.

## Revendications

1. Formulation pour pulvérisation d'épinéphrine destinée à être utilisée dans une méthode de traitement de l'anaphylaxie, ladite formulation comprenant :
d'environ 0,1 % à environ 15 % en p/p d'épinéphrine ou d'un sel de celle-ci ;
d'environ 1 % à environ 65 % en p/p d'acide chlorhydrique avec une normalité d'environ 0,1 à 12 N ;
d'environ 2 % à environ 60 % en p/p d'éthanol ;
d'environ 2 % à environ 98 % en p/p d'eau ;
d'environ 0,001 % à environ 0,5 % en p/p de bisulfite de sodium ;
d'environ 0,005 % à environ 1 % en p/p d'édétate disodique dihydraté et
d'environ 0,001 % à environ 0,5 % en p/p de chlorure de benzalkonium ; et
ladite formulation étant administrée par voie intranasale ;
p/p signifiant poids par poids de la formulation et « environ » étant défini comme étant ± 10 % de la valeur modifiée.

2. Formulation pour pulvérisation d'épinéphrine destinée à être utilisée selon la revendication 1, ladite formulation comprenant :
environ 3,2 % en p/p d'épinéphrine ou d'un sel de celle-ci ;
environ 35,5 % en p/p d'acide chlorhydrique avec une normalité de 0,5 ;
environ 40 % en p/p d'éthanol ;
environ 16,1 % en p/p d'eau ;
environ 0,05 % en p/p d'édétate disodique dihydraté ;
environ 0,15 % en p/p de bisulfite de sodium ;
environ 0,01 % en p/p de chlorure de benzalkonium.

3. Formulation pour pulvérisation d'épinéphrine destinée à être utilisée selon la revendication 2, ladite formulation comprenant en outre environ 5 % en p/p de propylène glycol.
